(19) **Europäisches Patentamt**
**European Patent Office**
**Office européen des brevets**

(11) **EP 3 929 165 B1**

(12) **EUROPEAN PATENT SPECIFICATION**

(45) Date of publication and mention
of the grant of the patent:
**17.04.2024 Bulletin 2024/16**

(21) Application number: **21180692.2**

(22) Date of filing: **21.06.2021**

(51) International Patent Classification (IPC):
**C03B 23/049** *(2006.01)*    **C03B 23/09** *(2006.01)*
**C03B 40/027** *(2006.01)*    **A61M 5/178** *(2006.01)*

(52) Cooperative Patent Classification (CPC):
**C03B 23/092; A61M 5/3129; C03B 23/0493;**
**C03B 40/027;** A61M 5/178; A61M 2207/00

(54) **APPARATUS FOR FORMING A CONE FOR HOUSING A NEEDLE IN A SYRINGE, METHOD FOR MAKING A CONE FOR HOUSING A NEEDLE IN A SYRINGE, AND THE SYRINGE THEREOF.**

VORRICHTUNG ZUR HERSTELLUNG EINES KEGELS ZUR UNTERBRINGUNG EINER NADEL IN EINER SPRITZE, VERFAHREN ZUR HERSTELLUNG EINES KEGELS ZUR UNTERBRINGUNG EINER NADEL IN EINER SPRITZE, UND DIE SPRITZE DAZU GEHÖRENDER SPRITZE.

APPAREIL PERMETTANT DE FORMER UN CONE POUR LOGER UNE AIGUILLE DANS UNE SERINGUE, PROCEDE DE FABRICATION D'UN CONE POUR LOGER UNE AIGUILLE DANS UNE SERINGUE, ET LA SERINGUE CORRESPONDANTE.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2020 IT 202000014869**

(43) Date of publication of application:
**29.12.2021 Bulletin 2021/52**

(73) Proprietor: **STEVANATO GROUP S.P.A.**
**35017 Piombino Dese (PD) (IT)**

(72) Inventors:
 • **CHILLON, Alberto**
   **I-35017 Piombino Dese, PADOVA (IT)**
 • **CHINELLATO, Fabio**
   **I-35017 Piombino Dese, PADOVA (IT)**
 • **NICOLETTI, Fabiano**
   **I-35017 Piombino Dese, PADOVA (IT)**

(74) Representative: **Long, Giorgio**
**Jacobacci & Partners S.p.A.**
**Piazza Mario Saggin, 2**
**35131 Padova (IT)**

(56) References cited:
**US-A1- 2019 144 326**

## Description

### FIELD OF APPLICATION

The present invention relates to a device for forming a cone for housing a needle in a syringe, a method for making the cone for housing a needle in a syringe, and a syringe thereof thus obtained.

### PRIOR ART

As is known, glass syringes comprising a hollow cylindrical syringe body, so as to accommodate a medical substance to be injected in solid, suspension, or solution form, are widely used in the medical industry. The injection is through a frontal delivery end via a needle applied therein, itself hollow and in fluid connection with the body cavity.

Internally housed in the cavity is a piston or plunger that is pushed by the user, or by automatic or semi-automatic systems, to allow the injection of a medical fluid, in a known manner.

The forming, on the syringe body, of the cone configured to fix the needle represents a critical step: in effect, the tip of the tool is attached to the syringe body just before closing the rollers and shaping the glass with said rollers. It is therefore necessary to keep the channel that will accommodate the portion of the needle that is attached to said syringe body open, i.e., pervious, in a stage wherein the glass is extremely hot and malleable: obviously this stage is critical because there is a risk that the hole for housing the needle may close easily.

In effect, this shaping must take place in a precise and controlled manner, since it could lead to the formation of cracks on the glass body, which is particularly fragile, and the consequent mechanical weakening. In addition, the hole for housing the needle must be formed with extreme precision to avoid residual or fragmented glass and/or sharp-edged portions that could result in subsequent cracks developing due to the subsequent insertion of the needle into the desired position or seat.

Moreover, it must be kept in mind that the operation of forming the cone for housing a needle in a syringe must also be as fast as possible, since batches of tens or hundreds of thousands of pieces must be processed. Obviously, increasing the forming speed poses greater risks in terms of defects.

Known solutions, in order to keep the housing hole pervious, involve the use of forming tips that comprise tungsten: tungsten is in effect a particularly hard material and resistant to high temperatures as well as to the considerable abrasion and wear that are generated on the tips when in contact with the glass to be formed.

The problem with tungsten is that, while it solves the problem of resistance to abrasion/wear/temperature by allowing a rather rapid forming operation, it is potentially a contaminant of the glass of the syringe. In other words, small amounts of tungsten from the tip are released onto the glass as a result of abrasion and redeposition of its salts and oxides generated by the high glass-forming temperatures. These tungsten derivatives are potentially incompatible with the medicinal substances and formulations contained in the syringe body and may over time alter their therapeutic efficacy.

For this reason, in forming processes using tungsten tips, said tips are used for a limited time (in the range of 2-4 hours) to minimize the release of tungsten compounds onto the syringe body. After that time, the tips must be replaced.

It is also known to use gas, typically nitrogen, blown during the forming stage in order to avoid or limit as much as possible the formation of tungsten oxides due to the oxidation action of the oxygen contained in the air.

However, these solutions are complex and increase the overall process costs.

Alternative forming tips that do not contain tungsten are also known, whereby the problem of formation of tungsten compounds, such as oxides and salts, is prevented upstream. Tips containing silicon nitride are known to be used for this purpose. However, these solutions have some drawbacks and disadvantages.

In effect, silicon nitride tips, although hard and resistant to medium-high temperatures, do not allow for the speeds and working temperatures reached by the equivalent (in terms of size and geometry) tungsten tips.

To at least partially overcome this limitation of temperature resistance, it is known to properly dose a flow of lubricant-coolant fluid at the contact between the glass and the forming tip, in order to lower its working temperature.

In any case, the lubricant-coolant fluid cannot always penetrate effectively and thus lubricate and cool the tip, because the absolute size, i.e., the thickness of the tip, is extremely small.

The result is that with such tips of the prior art, the productive performance of equivalent tungsten tips cannot be achieved or otherwise exceeded.

US2019144326 A1 discloses a method and an apparatus for shaping a glass workpiece such as a syringe with minimal lubrication.

### DISCLOSURE OF THE INVENTION

Thus, there is a need to resolve the cited drawbacks and limitations with reference to the prior art.

In particular, there is a need for providing forming tips for the housing cone of a syringe needle that allow precise, rapid, and reliable forming, that have a functional life not inferior to that of tungsten tips, and that ensure the total absence or strong reduction of the release of compounds on the syringe body, without requiring the costly and complex use of controlled atmospheres (e.g., with inert gas such as nitrogen).

This requirement is satisfied by a forming apparatus according to claim 1, a forming tool according to claim 10 and a method for making a cone for housing a needle in a syringe according to claim 13. A syringe made with the forming tool of claim 11 forms also part of the invention.

DESCRIPTION OF THE DRAWINGS

Further features and advantages of the present invention will be more readily understood from the following description of its preferred and non-limiting examples of embodiments, wherein:

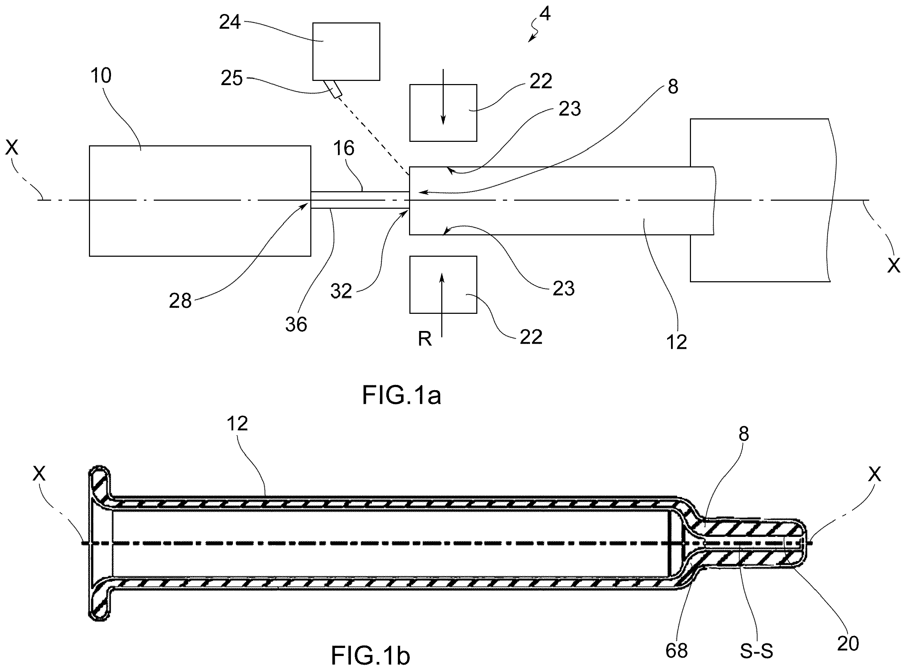

Fig. 1a depicts a side view of a forming apparatus according to one embodiment of the present invention;
Fig. 1b depicts a cross-sectional view of a syringe body formed according to the present invention;
Fig. 2a, 2b, 2c depict respectively two side views and a plan view from the tip side of a forming tool according to a possible embodiment of the present invention;
Fig. 3a, 3b, 3c depict respectively two side views and a plan view from the tip side of a forming tool according to a further possible embodiment of the present invention;
Fig. 4a, 4b, 4c depict respectively two side views and a plan view from the tip side of a forming tool according to a further possible embodiment of the present invention;
Fig. 5a, 5b, 5c depict respectively two side views and a plan view from the tip side of a forming tool according to a further possible embodiment of the present invention;
Fig. 6a, 6b, 6c depict respectively two side views and a plan view from the tip side of a forming tool according to a further possible embodiment of the present invention;
Fig. 7a, 7b, 7c depict respectively two side views and a plan view from the tip side of a forming tool according to a further possible embodiment of the present invention;
Fig. 8a, 8b, 8c depict respectively two side views and a plan view from the tip side of a forming tool according to a further possible embodiment of the present invention;
Fig. 9a, 9b, 9c depict respectively two side views and a plan view from the tip side of a forming tool according to a further possible embodiment of the present invention;
Fig. 10a, 10b, 10c depict respectively two side views and a plan view from the tip side of a forming tool according to a further possible embodiment of the present invention;
Fig. 11a, 11b, 11c depict respectively two side views and a plan view from the tip side of a forming tool according to a further possible embodiment of the present invention;
Fig. 12a, 12b, 12c depict respectively two side views and a plan view from the tip side of a forming tool according to a further possible embodiment of the present invention;
Fig. 13a, 13b, 13c depict respectively two side views and a plan view from the tip side of a forming tool according to a further possible embodiment of the present invention;
Fig. 14a, 14b, 14c depict respectively two side views and a plan view from the tip side of a forming tool according to a further possible embodiment of the present invention.

The elements or parts of elements in common among the embodiments described below will be indicated by the same numerical references.

DETAILED DESCRIPTION

With reference to the aforesaid figures, the numerical reference 4 has been used to refer globally to an apparatus for forming the housing cone 8 of a needle in a syringe body 12 of a glass syringe for medical substances. The syringe body 12 has a prevailing extension axis X-X.

The apparatus 4 comprises a forming tool 16 shaped to make a hole 20 for creating said housing cone 8 in the glass syringe body 12.

In other words, the housing cone 8 is obtained in successive stages by forming a wall of said glass syringe body 12 which is shrunk around said forming tool 16, preferably through the use of a pair of rollers 22. Therefore, the forming tool 16 acts as a male plug or pin while the side wall 23 of the syringe body 12 is shrunk thereon by said rollers 22 which move along a radial direction R-R, perpendicular to the prevailing extension axis X-X. Upon removal of the forming tool 16, there will then remain the hole 20 for later housing the needle of the syringe.

The forming apparatus 4 also comprises a lubricant-coolant liquid dispensing device 24 on the tip 32 of the forming tool 16 and/or in the contact area between said forming tool 16 and the glass syringe body 12. The dispensing device 24 may comprise one or more dispensing nozzles 25 of the lubricant-coolant liquid.

For the purposes of the present invention, neither the particular type of dispensing device 24 nor the type of lubricant-coolant liquid used is relevant.

The forming tool 16 comprises a grip or shank portion 28, suitable for gripping by relevant motor means. The motor means may translate, rotate, or roto-translate the forming tool 16 relative to the syringe body 12 along said prevailing extension axis X-X. It is also possible to hold the forming tool 16 stationary and rotate, translate, and/or roto-translate the syringe body 12 along said prevailing extension axis X-X. The forming tool 16 further comprises a tip 32 suitable for forming the syringe body 12, and a tip body 36, interposed between the tip 32 and the grip portion 28, suitable for making said hole 20 by machining the glass.

The grip portion 28, the tip body 36 and the tip 32 are preferably made in one piece with each other and are aligned along a prevailing extension axis as well as a rotation axis X-X of the forming tool 16.

Advantageously, the tip body 36, with respect to a cross-sectional plane perpendicular to the prevailing extension axis X-X, has a non-circular cross section, inscribed within the maximum circle 40 having as its radius the maximum distance between any point P of said cross section and the prevailing extension axis X-X, measured on said cross-sectional plane perpendicular to the prevailing extension axis X-X.

In other words, with respect to the cross-sectional plane perpendicular to the prevailing extension axis X-X, the maximum radius or distance from the same prevailing extension axis is considered, which defines the radius and thus the diameter of the hole 20 that may be made in the syringe body 12 after rotating the forming tool about its prevailing extension and rotation axis X-X.

This means that, with respect to a cross-sectional plane perpendicular to the prevailing extension axis X-X, the cross section of the tip body 36 will always be less than the cross section or area of the maximum circle 40, i.e., the circle having a radius equal to the radius of the hole 20 to be made. In still other words, the tip body 36 has one or more lateral excavations relative to said maximum circle 40.

Preferably, the tip body 36, at its maximum cross section along the prevailing extension axis X-X, has a cross section of less than 85% of the cross section of said maximum circle 40.

According to a further embodiment, the tip body 36, at its maximum cross section along the prevailing extension axis X-X, has a cross section of less than 70% of the cross section of said maximum circle 40.

It is possible to further reduce the ratio between the tip body cross section 36 and the maximum circle 40, so that the necessary mechanical torsional (but also bending) strength of the tip body 36 is always ensured.

In particular, the cross section of the tip body 36 defines, relative to the maximum circle 40, at least one recess 44 suitable to allow the passage of said lubricant-coolant liquid.

In other words, with respect to the theoretical maximum size of the tip body 36 given by the maximum circle 40, it is envisaged to use a geometry that is not circular but instead has at least one recess 44 that constitutes a cavity adapted to form a passage for the lubricant-coolant liquid.

According to one embodiment, the tip body 36, at a cross section along the prevailing extension axis X-X, has a plurality of recesses 44 fluidly connected to each other so as to create a continuous channel for the passage of said lubricant-coolant liquid.

Preferably, the recesses 44 of the tip body 36 are in fluid connection with each other along the prevailing extension axis X-X, so as to create a continuous channel for the passage of said lubricant-coolant liquid along the tip body 36.

The cross section of the tip body 36 is preferably constant along the prevailing extension axis: in other words, the tip body 36 is cylindrical, i.e., consisting of straight lines all parallel to the prevailing extension axis, but having a cross section different from the circular cross section (in particular, smaller than the maximum circle 40 due to the presence of at least one recess 44).

Obviously, for the purposes of mechanical strength and durability/reliability of the forming tool 16, it is preferable for the cross section of the syringe body 36 to be as symmetrical as possible.

It is also possible for the cross section of the tip body 36 to vary along said prevailing extension axis X-X.

For example, according to one possible embodiment, the cross section of the tip body 36 tapers along said prevailing extension axis X-X, moving from the shank or grip portion 28 toward the tip 32.

There are many possible geometries for the tip body 36.

For example, said cross section of the tip body 36 may be a regular polygon, inscribed within said maximum circle 40, such as a triangle, a square, a rhombus, a pentagon, a hexagon, and so forth.

It is also possible for the cross section of the tip body 36 to be a closed polyline, inscribed within said maximum circumference, as in the case of a rectangle, trapezoid, or any closed geometry.

It is also possible to make a cross section of the tip body 36 that is curvilinear, inscribed within said maximum circle 40. This cross section may have straight sides and/or curved sides and so forth.

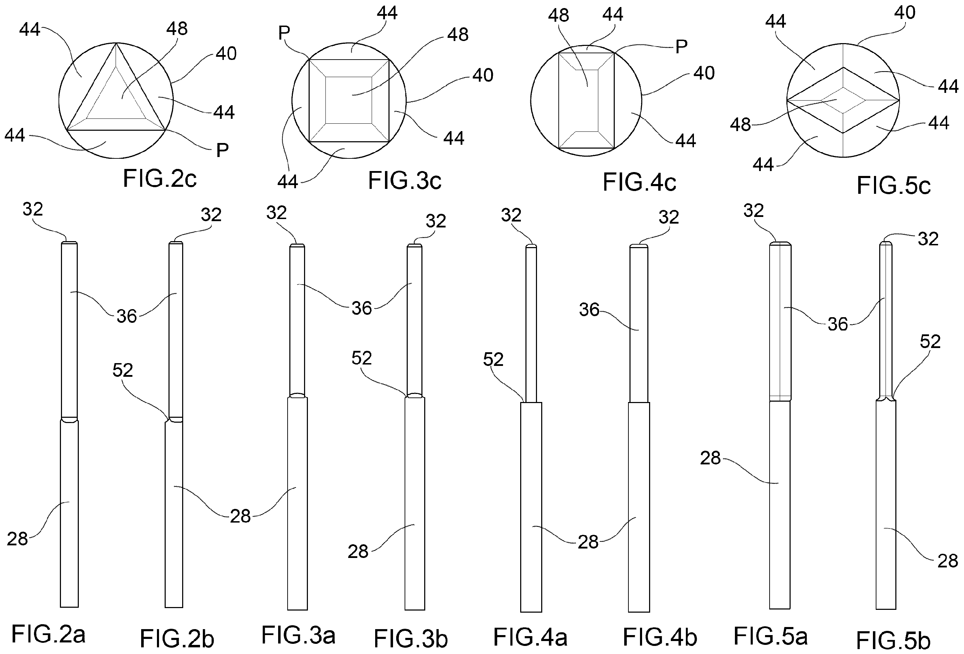

Fig. 2-14 depict some of these possible embodiments according to the present invention.

As shown by way of example, Fig. 2-14 show some of the possible geometries of the tip body 36 according to variant embodiments of the present invention.

For example, in Fig. 2a-2c, a triangular cross-sectional geometry is envisaged, specifically according to an equilateral triangle inscribed in the maximum circle 40.

Fig. 3a-3c illustrate a square cross-sectional geometry; Fig. 4a-4c illustrate a rectangular cross-sectional geometry; Fig. 5a-5c illustrate a rhomboidal cross-sectional geometry.

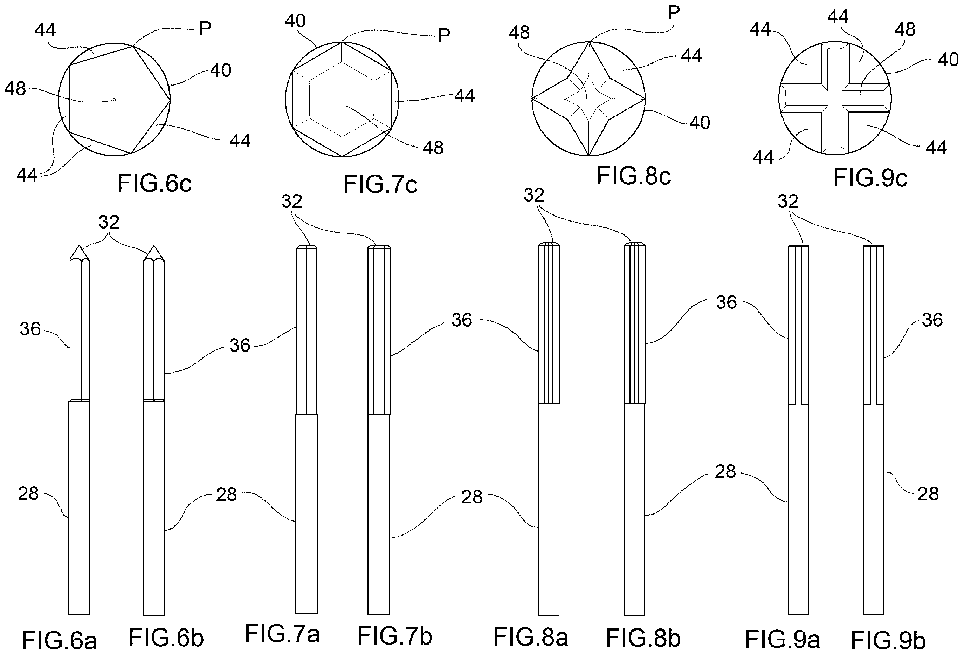

A pentagonal cross-sectional geometry is envisaged in Fig. 6a-6c, while a hexagonal cross-sectional geometry is envisaged in Fig. 7a-7c.

Fig. 8a-8c illustrate a star cross-sectional geometry, while Fig. 9a-9c envisage a cross-shaped cross-sectional geometry, with arms perpendicular and equal to each other, with a length equal to the diameter of the maximum circle 40.

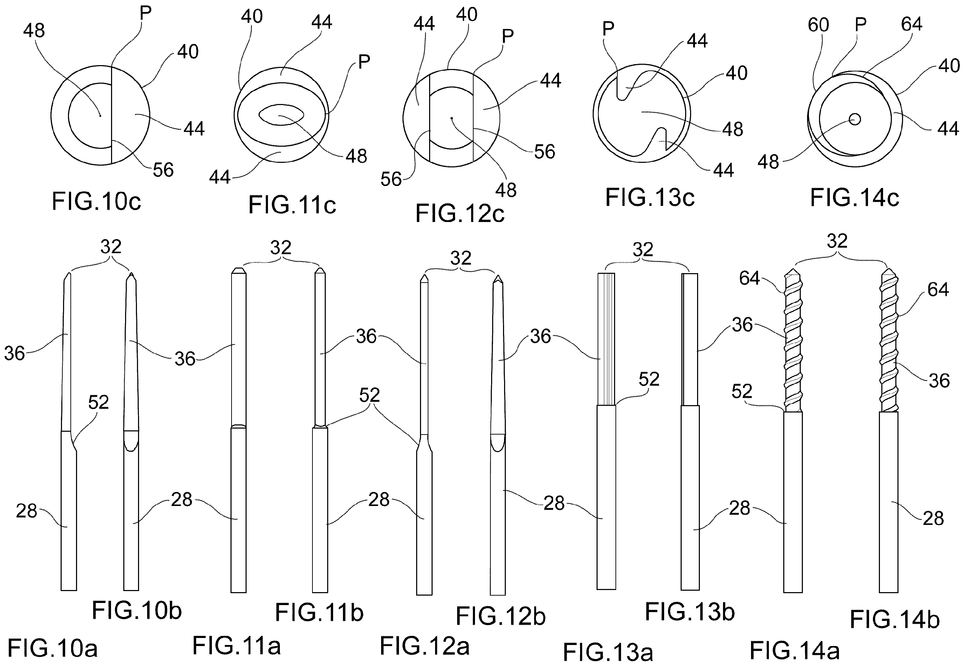

In Fig. 10a-10c, a partially circular cross-sectional geometry is envisaged, provided with a facet 56 on one side; preferably, but not exclusively, said facet 56 has an extension less than the diameter of the remaining circular cross section.

In Fig. 11a-11c, an elliptical cross-sectional geometry is envisaged, wherein the major axis of the ellipse is equal to the diameter of the maximum circle 40.

In Fig. 12a-12c, a partially circular cross-sectional geometry is envisaged, provided with a pair of facets 56 arranged on opposite, and preferably symmetrical, sides with respect to the prevailing extension axis X-X.

Fig. 13a-13c illustrate a circular cross-sectional geometry, with a diameter equal to the diameter of the maximum circle 40, provided with a pair of recesses 44, with a substantially parabolic geometry, arranged on opposite sides relative to the prevailing extension axis X-X.

Lastly, Fig. 14a-14c illustrate a circular cross-sectional geometry, having a diameter less than the diameter of the maximum circle 40, provided with a circular arc 60 substantially tangent to the diameter of said maximum circle 40.

Said circular arc corresponds to a thread 64 that screws as a helix about said circular geometry, along the prevailing extension axis X-X.

The tip 32 is preferably tapered relative to its attachment portion to the tip body 36.

Preferably, said tip 32 has the same geometry as the tip body 36, with respect to a cross-sectional plane perpendicular to the prevailing extension axis X-X.

For example, if the tip body 36 has a square cross section, the tip 32 will also have a square cross section, but tapered, i.e., with a smaller side.

The tip 32 does not need to have sharp ends 48.

For example, the tip may have a flat end 48 contained in a plane perpendicular to the prevailing extension axis X-X.

It is also possible to provide said tip 32 with a conical, pyramidal, or frustoconical end 48.

According to one embodiment, a step or neck-in 52 is provided, relative to a cross-sectional plane perpendicular to the prevailing extension axis X-X, at an area for attaching the grip or shank portion 28 to the tip body 36.

The shank 28 may have any cross section. The shank 28 may even have a circular cross section even equal to said maximum circle 40.

The function of the shank 28 is to allow grasping and/or movement of the forming tool 16 about the prevailing extension axis X-X but does not have the function of removing material from the syringe body 12.

Preferably, the grip or shank portion 28, the tip 32, and the tip body 36 are made of metallic, and/or ceramic, and/or non-metallic material and are tungsten-free.

Obviously, it is also possible to apply the present invention to tips made wholly, or even partially, of tungsten.

As mentioned above, the particular geometry of the forming tool 16 and the tip 32 allows for lubrication and temperature containment at the housing cone 8 whereby contamination of the cone with tungsten compounds (which would easily form at the high temperatures reached with conventional tips) is reduced or the use of ceramic tips with a certain level of breakage resistance is permitted.

However, it was found that by using a ceramic material doped with an yttrium compound, a forming tool 16 with a high-strength tip 32 may be obtained. In particular, the combination of this ceramic material doped with yttrium compounds with the geometry of the forming tool 16 and of the tip 32 previously described makes it possible to avoid the use of tungsten tips without losing the characteristics of resistance to high temperatures typical of tungsten tips.

In preferred embodiments, the ceramic material used is silicon nitride ($Si_3N_4$) doped with yttrium oxide ($Y_2O_3$), wherein more preferably the yttrium oxide is contained in the silicon nitride in amounts between 3% and 7% or between 4% and 6% by weight. In certain embodiments, the silicon nitride contains yttrium oxide and alumina ($Al_2O_3$) in a combined amount of between 7% and 13% or between 8% and 12% by weight.

In order to evaluate the residual amount of yttrium present in the cone of a syringe after it has been formed with said forming tool 16 from ceramic material doped with an yttrium compound, analytical detection methods have been developed and are described below.

An analytical method of detecting yttrium is as follows.

Measurement: element Y removable from the syringe cone by extraction in heated ultrasonic bath, with 2% nitric acid (2% $HNO_3$) as extracting solvent;
Analytical technique: Inductively Coupled Plasma-Mass Spectrometry (ICP-MS);
Test item: bulk syringes (not assembled with needle, lacking internal coating), in neutral borosilicate Type I glass (as defined in the USP standard <660>, neutral borosilicate Type I glass), with two types of cone format (Staked Needle - SN - and Luer Lock - LLC);
Quantification Range: 0.1-200 pg/L;
Extractive methodology:

a) Insert each syringe into a screw-capped test tube
b) Fill the syringe with 1 ml of 2% $HNO_3$ and close the tube with its cap
c) Immerse in preheated ultrasonic bath for 1 h at 75°C
d) Shake (with vortex shaker) the test tube with the syringe therein
e) Allow to cool to room temperature
f) Remove the syringe from the test tube taking care to empty all the liquid in the test tube
g) Take 0.3 ml of the extracted solution, transfer it to a new clean test tube, and add 2.7 ml of internal standard solution (Iridium 56 pg/L in 2% $HNO_3$), thus diluting the extract by a factor of 10

h) Shake the solution to homogenize it.

Instrument set-up:

- Volume of sample submitted for analysis: 3 mL (obtained by dilution as in step (g))
- Use of Internal Standards: Iridium, at a final concentration of 50 pg/L
- Mode of acquisition: Standard
- Instrument conditioned in 2% $HNO_3$ and calibrated with calibration line for the element Y in the range 0.1-200 pg/L
- Atomic mass: 193Ir, 89Y;

Calculation of the amount of Y extracted per syringe (considering the extraction volume of 1 mL):

$$Y\ (ng/syringe) = C * FD$$

Where C = concentration in the diluted extract (expressed in pg/L) returned by the software
FD = dilution factor (equal to 10)

A second method of yttrium extraction from the cone 8 is described below.

Measurement: element Y present in the syringe cone quantifiable following total mineralization/ digestion of the glass matrix (only cone area fragment) with the aid of hydrofluoric acid or other solvents propaedeutic to mineralization.

Analytical technique: Inductively Coupled Plasma-Mass Spectrometry (ICP-MS)
Test item: bulk syringes (not assembled with needle, lacking internal coating), in neutral borosilicate Type I glass (as defined in USP <660>, neutral borosilicate Type I glass), with two types of cone format (Staked Needle - SN - and Luer Lock - LLC);
A third method of yttrium extraction from the cone 8 is described below.

Measurement: element Y present in the syringe cone identifiable by fragmentation of the syringe cone area, subjected to laser ablation for sampling and subsequent determination by ICP-MS without the need for pretreatment or derivatization.

Analytical technique: Laser ablation, Inductively Coupled Plasma Mass Spectrometry (LA-ICP-MS)

Test item: bulk syringes (not assembled with needle, lacking internal coating), in neutral borosilicate Type I glass (as defined in USP <660>, neutral borosilicate Type I glass), with two types of cone format (Staked Needle - SN - and Luer Lock - LLC);
Using the methods described above, it has been determined that the yttrium content in the cone of syringes formed with the tip and according to the method of the present invention is between 0.5 ng and 1 ng.

The forming apparatus 4 may include at least one second forming tool shaped for finishing said hole 20 created by means of the forming tool 16.

In other words, the forming apparatus 4 often comprises a plurality of forming tools 16 that have the function of creating the shape of the needle housing cone 8 by successive stages or steps: the first stage consists in the creation of roughing in or formation of the main hole, while subsequent stages are used to define the details. The forming tools 16 used may have the features described above. It is possible that said forming tools 16 have the same shapes but different sizes or that they also have different shapes/geometries/materials. A plurality of forming tools constitute a set of forming tools 16.

The operation or method for forming a cone for housing a needle in a syringe according to the present invention will now be described.

In particular, the forming tool 16 is mounted on a suitable drive means through the shank 28.

Next, the syringe body 12 is rotated about the prevailing extension and rotation axis X-X, taking care to also activate the flow of lubricant-coolant liquid in the area of the tip 32 and/or the tip body 36.

Due to the geometry of the tip body 36, which envisages a smaller cross section than that of the maximum circle 40, the lubricant-coolant liquid allows the glass of the syringe body 12 to be machined, creating the hole 20 and, at the same time, allowing an adequate flow of lubricant-coolant liquid to pass through in order to avoid overheating the forming tool 16 and thus premature wear.

Obviously, the center of the hole 20 (to be made) in the syringe body 12 must be centered or aligned with said prevailing extension axis X-X.

After making the hole 20, the forming tool 16 is extracted and the finishing of the surface of said hole 20 is continued through the use of at least a second forming tool, as described above.

As may be appreciated from that which has been described, the apparatus for forming a cone for housing a needle in a syringe according to the invention enables the drawbacks presented in the prior art to be overcome.

In particular, the present invention makes it possible to avoid or otherwise significantly reduce the release of tungsten compounds on the glass syringe body since it envisages the use of a forming tip that may be entirely tungsten-free.

Thus, the present invention enables a transition from a glass syringe having a low tungsten content (as per solutions of the prior art that make use of tungsten tips and employ techniques to contain the release of tungsten on the glass body), to a glass syringe that is entirely free of tungsten or otherwise contains entirely lower and negligible amounts of tungsten compounds than in the solutions of the prior art.

Despite the absence of tungsten, the forming tip of the present invention allows the same forming precision and speed achievable with a tungsten tip to be obtained, without the use of any controlled atmosphere.

In effect, on the one hand, the absence of tungsten prevents the formation of relevant tungsten compounds and on the other, envisaging a special geometry of the tip body allows the use of an abundant flow of lubricant-coolant fluid so as to effectively control and contain the heating temperature of the tip.

Due to this geometry, there is an adequate cross-sectional passage for the lubricant-coolant fluid that may effectively reach the areas most stressed from a mechanical and thermal point of view, avoiding both the onset of excessive heating and processing imperfections that could generate future cracks in the glass. For example, it is possible to eliminate the so-called "screwing" of the syringe cone surface typical of the methods of the prior art, due to the suboptimal flow of the coolant; this effect manifests itself in an irregular and, in particular, wavy profile of the inner profile of the syringe of the cone; in the case of conventional tips, this effect may in fact be reduced, but not eliminated, only by slowing down the rotation speed, thus slowing down the process consequently.

The mechanical wear of the forming tip of the present invention may also be advantageously monitored and kept under control since the tip is constantly and effectively lubricated and cooled during mechanical processing on the glass body. Therefore, even when using materials less resistant than tungsten, such as silicon nitrides, the tips will still have a reduced consumption and may ensure a high machining precision, since they will never reach critical temperatures due to the presence of effective cooling and lubrication during the mechanical processing of the glass.

In particular, in the case of using a forming tool 16 made of ceramic material doped with an yttrium compound, it is possible to obtain syringes totally free of tungsten (and with an irrelevant residual yttrium content), optimizing at the same time the production process, i.e., providing tips 32 with high strength and durability, which is not possible with ceramic tips of conventional shape and composition.

These syringes are particularly adapted for certain active ingredients sensitive to the presence of tungsten, such as in particular: tocilizumab, Darbepoietin alfa, bevacizumab, Interferon beta 1-alfa, interferon beta 1b, onabotulinum toxin A, exenatide, imiglucerase, certolizumab pegol, glatiramer acetate, secukinumab, triptorelin, dupilumab, etanercept, epoetin, cetuximab, aflibercept, follitropin beta, teriparatide, papilloma virus vaccines, glucagon, FSH - follicolum stimulating hormone, trastuzumab, insulin lispro, Insulin, adalimumab, dibotermin alfa, interferon alfa 2a, paliperidone, pembrolizumab, anakinra, factor VIII - Antiemophilic factor, insulin glargine, enoxaparin, ranibizumab, alemtuzumab, rituximab, tenecteplase, botulinum toxin type A, epoetina beta, pegfilgrastim, filgrastim, somatropin, insulin aspart, activated eptagon alfa, romiplostim, peg aspargase, nivolumab, abatacept, choriogonadotropin alfa, pegilated interferon alfa 2a, pertuzumab, pegilated inteferon beta 1a, streptococcus pneumonia vaccines, denosumab, infliximab, alteplasa, golimumab, basiliximab, eculizumab, ustekinumab, palivizumab, atezolizumab, insulin degludec, ibalizumab, liraglutide, ranibizumab, omalizulab, and pegaspargase.

It is therefore a further subject matter of the present invention to have a syringe made with the forming tool of claim 11 filled with one of the aforementioned active substances, characterized in that said syringe does not contain tungsten, and contains a residual amount of yttrium between 0.5 ng and 1 ng.

## Claims

1. A forming apparatus (4) of the housing cone (8) of a needle in a syringe body (12) of a glass syringe for medical substances, comprising

   - a forming tool (16) shaped to make a hole (20) for the creation of said housing cone (8) in the glass syringe body (12),
   - at least one dispensing device (24) of lubricant-coolant liquid in the contact zone between said forming tool (16) and the glass syringe body (12),

   wherein the forming tool (16) comprises

   - a grip or shank portion (28), suitable to be gripped and moved by means of relevant motor means,
   - a tip (32) suitable for forming said hole (20) on the syringe body (12),
   - a tip body (36), between the tip (32) and the grip portion (28), suitable for making said hole (20),
   - the grip portion (28), the tip body (36) and the tip (32) being in one piece and aligned along a prevailing extension and rotation axis (X-X) of the syringe body (12) and/or the forming tool (16),

   **characterized in that**

   - the tip body (36), with respect to a cross-sectional plane perpendicular to the prevailing extension axis (X-X), has a non-circular cross section, inscribed within the maximum circle (40), having as its radius the maximum distance between a point (P) of said cross section and the prevailing extension axis (X-X), measured on said cross-sectional plane perpendicular to the prevailing extension axis (X-X), so as to have an overall cross section smaller than the cross section of the maximum circle (40).

2. The forming apparatus (4) according to claim 1, wherein the tip body (36), at its maximum cross section along the prevailing extension axis X-X, has a cross section less than 85% of the cross section of said maximum circle (40) and wherein the tip body (36), at its maximum cross section along the prevailing extension axis X-X, has a cross section less than 70% of the cross section of said maximum circle (40).

3. The forming apparatus (4) according to any of the claims from 1 to 2, wherein the cross section of the tip body (36) identifies, with respect to the maximum circle (40), at least one recess (44) suitable to allow the passage of said lubricant-coolant liquid.

4. The forming apparatus (4) according to claim 3, wherein the tip body (36) presents, at a cross section along the prevailing extension axis (X-X), a plurality of recesses (44) fluidically connected to each other so as to create a continuous channel for the passage of said lubricant-coolant liquid.

5. The forming apparatus (4) according to any of the claims from 3 to 4, wherein the recesses (44) of the tip body (36) are connected to each other along the prevailing extension axis (X-X), so as to create a continuous channel for the passage of said lubricant-coolant liquid along the tip body (36).

6. The forming apparatus (4) according to any of the claims from 1 to 5, wherein said cross section of the tip body (36) varies along said prevailing extension axis (X-X) and wherein the cross section of the tip body (36) tapers along said prevailing extension axis (X-X), moving from the shank (28) towards the tip (32).

7. The forming apparatus (4) according to any of the claims from 1 to 6, wherein said cross section of the tip body (36) is a regular polygon, inscribed inside said maximum circle (40), wherein said cross section of the tip body (36) is a closed polyline, inscribed inside said maximum circle (40) and wherein the cross section of the tip body (36) is curved, inscribed inside said maximum circle (40).

8. The forming apparatus (4) according to any of the claims from 1 to 7, wherein said tip (32) is tapered with respect to an attachment portion thereof to the tip body (36).

9. The forming apparatus (4) according to any of the claims from 1 to 8, wherein said tip (32) has the same geometry as the tip body (36), with respect to a cross-sectional plane perpendicular to the prevailing extension axis (X-X).

10. A forming tool (16) shaped to make a hole (20) for creating a housing cone (8) in the body of a glass syringe (12), wherein said forming tool (16) comprises

   - a grip or shank portion (28), suitable to be gripped and moved by means of relevant motor means,
   - a tip (32) suitable for forming said hole (20) on the syringe body (12),
   - a tip body (36), between the tip (32) and the grip portion (28), suitable for making said hole (20),
   - the grip portion (28), the tip body (36) and the tip (32) being in one piece and aligned along a prevailing extension and rotation axis (X-X) of the syringe body (12) and/or the forming tool (16),

   **characterized in that**

   - the tip body (36), with respect to a cross-sectional plane perpendicular to the prevailing extension axis (X-X), has a non-circular cross section, inscribed within the maximum circle (40), having as its radius the maximum distance between a point (P) of said cross section and the prevailing extension axis (X-X), measured on said cross-sectional plane perpendicular to the prevailing extension axis (X-X), so as to have an overall cross section smaller than the cross section of the maximum circle (40).

11. The forming tool according to claim 10, said forming tool (16), and in particular the tip (32) and the tip body (36) of said tool being made of a ceramic material doped with an yttrium compound.

12. The forming tool (16) according to claim 11, wherein said ceramic material is silicon nitride ($Si_3N_4$) doped with yttrium oxide ($Y_2O_3$), wherein preferably the yttrium oxide is contained in the silicon nitride in amounts between 3% and 7% or between 4% and 6% by weight, or said ceramic material is silicon nitride containing yttrium oxide and alumina ($Al_2O_3$) in a combined amount of between 7% and 13%, or between 8% and 12% by weight.

13. A method for making a glass syringe for medical substances, provided with a housing cone (8) of a needle, comprising the steps of:

   - preparing a glass syringe body (12) provided with a side wall (23) intended to delimit said hole (20) in the housing cone (8),

- providing a forming tool (4) according to any of the claims from 1 to 9,
- rotating the syringe body (12) and/or the forming tool (16) about the prevailing extension axis (X-X),
- forming said side wall (23) of the syringe body (12), after aligning the prevailing extension axis (X-X) of the forming tool (16) with an axis of symmetry (S-S) of the hole (20) to be made.

14. A syringe made with the forming tool (16) according to claim 11, **characterized in that** said syringe (12) does not contain tungsten and contains a residual amount of yttrium between 0.5 ng and 1 ng.

15. A syringe (12) according to claim 14, filled with one of the following active substances: tocilizumab, Darbepoetin alfa, bevacizumab, Interferon beta 1-alpha, interferon beta 1b, onabotulinum toxin A, exenatide, imiglucerase, certolizumab pegol, glatiramer acetate, secukinumab, triptorelin, dupilumab, etanercept, epoetin, cetuximab, aflibercept, follitropin beta, teriparatide, papilloma virus vaccines, glucagon, FSH - folliculum stimulating hormone, trastuzumab, insulin lispro, Insulin, adalimumab, dibotermin alfa, interferon alfa 2a, paliperidone, pembrolizumab, anakinra, factor VIII - Antiemophilic factor, insulin glargine, enoxaparin, ranibizumab, alemtuzumab, rituximab, tenecteplase, botulinum toxin type A, epoetin beta, pegfilgrastim, filgrastim, somatropin, insulin aspart, activated heptagon alfa, romiplostim, peg aspargase, nivolumab, abatacept, choriogonadotropin alfa, pegilated interferon alfa 2a, pertuzumab, pegilated intefereon beta 1a, streptococcus pneumonia vaccines, denosumab, infliximab, alteplasa, golimumab, basiliximab, eculizumab, ustekinumab, palivizumab, atezolizumab, insulin degludec, ibalizumab, liraglutide, ranibizumab, omalizulab and pegaspargase.

**Patentansprüche**

1. Formvorrichtung (4) des Gehäusekonus (8) einer Nadel in einem Spritzenkörper (12) einer Glasspritze für medizinische Substanzen, umfassend:

- ein Formwerkzeug (16), das so geformt ist, dass es ein Loch (20) zum Herstellen des Gehäusekonus (8) in dem Glasspritzenkörper (12) erzeugt,
- zumindest eine Abgabevorrichtung (24) für Schmiermittel-Kühlflüssigkeit in der Kontaktzone zwischen dem Formwerkzeug (16) und dem Glasspritzenkörper (12),

wobei das Formwerkzeug (16) umfasst

- einen Griff- oder Schaftabschnitt (28), der geeignet ist, mittels relevanter Motormittel gegriffen und bewegt zu werden,
- eine Spitze (32), die geeignet ist, das Loch (20) an dem Spritzenkörper (12) zu bilden,
- einen Spitzenkörper (36) zwischen der Spitze (32) und dem Griffabschnitt (28), der zum Herstellen des Lochs (20) geeignet ist,
- wobei der Griffabschnitt (28), der Spitzenkörper (36) und die Spitze (32) einstückig sind und entlang einer vorherrschenden Erstreckungs- und Drehachse (X-X) des Spritzenkörpers (12) und/oder des Formwerkzeugs (16) ausgerichtet sind,

**dadurch gekennzeichnet, dass**

- der Spitzenkörper (36) in Bezug auf eine Querschnittsebene senkrecht zu der vorherrschenden Erstreckungsachse (X-X) einen nicht kreisförmigen Querschnitt aufweist, der in den maximalen Kreis (40) eingeschrieben ist, der als seinen Radius den maximalen Abstand zwischen einem Punkt (P) des Querschnitts und der vorherrschenden Erstreckungsachse (X-X) aufweist, und zwar gemessen an bzw. auf der Querschnittsebene senkrecht zu der vorherrschenden Erstreckungsachse (X-X), um einen Gesamtquerschnitt aufzuweisen, der kleiner als der Querschnitt des maximalen Kreises (40) ist.

2. Formvorrichtung (4) nach Anspruch 1, wobei der Spitzenkörper (36) bei seinem maximalen Querschnitt entlang der vorherrschenden Erstreckungsachse X-X einen Querschnitt von weniger als 85 % des Querschnitts des maximalen Kreises aufweist (40) und wobei der Spitzenkörper (36) bei seinem maximalen Querschnitt entlang der vorherrschenden Erstreckungsachse X-X einen Querschnitt von weniger als 70 % des Querschnitts des maximalen Kreises (40) aufweist.

3. Formvorrichtung (4) nach einem der Ansprüche 1 bis 2, wobei der Querschnitt des Spitzenkörpers (36) in Bezug auf den maximalen Kreis (40) zumindest eine Aussparung (44) identifiziert, die geeignet ist, den Durchgang der Schmiermittel-Kühlflüssigkeit zu erlauben.

4. Formvorrichtung (4) nach Anspruch 3, wobei der Spitzenkörper (36) an einem Querschnitt entlang der vorherrschenden Erstreckungsachse (X-X) eine Mehrzahl von Aussparungen (44) darstellt bzw. aufweist, die fluidisch miteinander verbunden sind, um einen kontinuierlichen bzw. durchgehenden Kanal für den Durchgang der Schmiermittel-Kühlflüssigkeit herzustellen.

**5.** Formvorrichtung (4) nach einem der Ansprüche 3 bis 4, wobei die Aussparungen (44) des Spitzenkörpers (36) entlang der vorherrschenden Erstreckungsachse (X-X) miteinander verbunden sind, um einen kontinuierlichen bzw. durchgehenden Kanal für den Durchgang der Schmiermittel-Kühlflüssigkeit entlang des Spitzenkörpers (36) herzustellen.

**6.** Formvorrichtung (4) nach einem der Ansprüche 1 bis 5, wobei der Querschnitt des Spitzenkörpers (36) entlang der vorherrschenden Erstreckungsachse (X-X) variiert und wobei sich der Querschnitt des Spitzenkörpers (36) entlang der vorherrschenden Erstreckungsachse (X-X) verjüngt, wobei er sich von dem Schaft (28) zu der Spitze (32) bewegt.

**7.** Formvorrichtung (4) nach einem der Ansprüche 1 bis 6, wobei der Querschnitt des Spitzenkörpers (36) ein regelmäßiges Polygon ist, das in den maximalen Kreis (40) eingeschrieben ist, wobei der Querschnitt des Spitzenkörpers (36) eine geschlossene Polylinie ist, die in den maximalen Kreis (40) eingeschrieben ist, und wobei der Querschnitt des Spitzenkörpers (36) gekrümmt ist und in den maximalen Kreis (40) eingeschrieben ist.

**8.** Formvorrichtung (4) nach einem der Ansprüche 1 bis 7, wobei die Spitze (32) in Bezug auf einen Anbringungsabschnitt derselben an dem Spitzenkörper (36) verjüngt ist.

**9.** Formvorrichtung (4) nach einem der Ansprüche 1 bis 8, wobei die Spitze (32) in Bezug auf eine Querschnittsebene senkrecht zu der vorherrschenden Erstreckungsachse (X-X) die gleiche Geometrie wie der Spitzenkörper (36) aufweist.

**10.** Formwerkzeug (16), das so geformt ist, dass es ein Loch (20) zum Herstellen eines Gehäusekonus (8) in dem Körper einer Glasspritze (12) erzeugt, wobei das Formwerkzeug (16) umfasst:

- einen Griff- oder Schaftabschnitt (28), der geeignet ist, mittels relevanter Motormittel gegriffen und bewegt zu werden,
- eine Spitze (32), die geeignet ist, das Loch (20) an dem Spritzenkörper (12) zu bilden,
- einen Spitzenkörper (36) zwischen der Spitze (32) und dem Griffabschnitt (28), der zum Herstellen des Lochs (20) geeignet ist,
- wobei der Griffabschnitt (28), der Spitzenkörper (36) und die Spitze (32) einstückig sind und entlang einer vorherrschenden Erstreckungs- und Drehachse (X-X) des Spritzenkörpers (12) und/oder des Formwerkzeugs (16) ausgerichtet sind,

**dadurch gekennzeichnet, dass**

- der Spitzenkörper (36) in Bezug auf eine Querschnittsebene senkrecht zu der vorherrschenden Erstreckungsachse (X-X) einen nicht kreisförmigen Querschnitt aufweist, der in den maximalen Kreis (40) eingeschrieben ist, der als seinen Radius den maximalen Abstand zwischen einem Punkt (P) des Querschnitts und der vorherrschenden Erstreckungsachse (X-X) aufweist, und zwar gemessen an bzw. auf der Querschnittsebene senkrecht zu der vorherrschenden Erstreckungsachse (X-X), um einen Gesamtquerschnitt aufzuweisen, der kleiner als der Querschnitt des maximalen Kreises (40) ist.

**11.** Formwerkzeug nach Anspruch 10, wobei das Formwerkzeug (16) und insbesondere die Spitze (32) und der Spitzenkörper (36) des Werkzeugs aus einem mit einer Yttriumverbindung dotierten Keramikmaterial bestehen.

**12.** Formwerkzeug (16) nach Anspruch 11, wobei das Keramikmaterial mit Yttriumoxid ($Y_2O_3$) dotiertes Siliziumnitrid ($Si_3N_4$) ist, wobei das Yttriumoxid vorzugsweise in Mengen zwischen 3 Gew.-% und 7 Gew.-% oder zwischen 4 Gew.-% und 6 Gew.-% in dem Siliziumnitrid enthalten ist, oder das Keramikmaterial Siliziumnitrid ist, das Yttriumoxid und Aluminiumoxid ($Al_2O_3$) in einer kombinierten Menge zwischen 7 Gew.-% und 13 Gew.-% oder zwischen 8 Gew.-% und 12 Gew.-% enthält.

**13.** Verfahren zum Herstellen einer Glasspritze für medizinische Substanzen, die mit einem Gehäusekonus (8) einer Nadel versehen ist, umfassend die Schritte:

- Vorbereiten eines Spritzenkörpers (12) aus Glas, der mit einer Seitenwand (23) versehen ist, die das Loch (20) in dem Gehäusekonus (8) begrenzen soll,
- Bereitstellen eines Formwerkzeugs (4) nach einem der Ansprüche 1 bis 9,
- Drehen des Spritzenkörpers (12) und/oder des Formwerkzeugs (16) um die vorherrschende Erstreckungsachse (X-X),
- Formen der Seitenwand (23) des Spritzenkörpers (12), nachdem die vorherrschende Erstreckungsachse (X-X) des Formwerkzeugs (16) mit einer Symmetrieachse (S-S) des herzustellenden Lochs (20) ausgerichtet wurde.

**14.** Spritze, die mit dem Formwerkzeug (16) nach Anspruch 11 hergestellt wird, **dadurch gekennzeichnet, dass** die Spritze (12) kein Wolfram enthält und eine Restmenge an Yttrium zwischen 0,5 ng und 1 ng enthält.

**15.** Spritze (12) nach Anspruch 14, gefüllt mit einem der folgenden Wirkstoffe: Tocilizumab, Darbepoetin alfa, Bevacizumab, Interferon beta 1-alpha, Interferon beta 1b, Onabotulinumtoxin A, Exenatide, Imiglucerase, Certolizumab Pegol, Glatiramer Acetat, Secukinumab, Triptorelin, Dupilumab, Etanercept, Epoetin, Cetuximab, Aflibercept, Follitropin beta, Teriparatid, Papillomavirus-Impfstoffe, Glucagon, FSH - Follikel-stimulierendes Hormon, Trastuzumab, Insulin lispro, Insulin, Adalimumab, Dibotermin alfa, Interferon alfa 2a, Paliperidon, Pembrolizumab, Anakinra, Faktor VIII - Antiemophiler Faktor, Insulin Glargin, Enoxaparin, Ranibizumab, Alemtuzumab, Rituximab, Tenecteplase, Botulinumtoxin Typ A, Epoetin Beta, Pegfilgrastim, Filgrastim, Somatropin, Insulin Aspart, aktiviertes Heptagon alfa, Romiplostim, Peg Aspargase, Nivolumab, Abatacept, Choriogonadotropin alfa, pegiliertes Interferon alfa 2a, Pertuzumab, pegiliertes Intefereon beta 1a, Streptokokken-Pneumonie-Impfstoffe, Denosumab, Infliximab, Alteplasa, Golimumab, Basiliximab, Eculizumab, Ustekinumab, Palivizumab, Atezolizumab, Insulin Degludec, Ibalizumab, Liraglutide, Ranibizumab, Omalizulab und Pegaspargase.

## Revendications

1. Appareil de formage (4) du cône de logement (8) d'une aiguille dans un corps de seringue (12) d'une seringue en verre pour substances médicales, comprenant

    - un outil de formage (16) profilé pour réaliser un trou (20) permettant la création dudit cône de logement (8) dans le corps de seringue en verre (12),
    - au moins un dispositif de distribution (24) de liquide lubrifiant-réfrigérant dans la zone de contact entre ledit outil de formage (16) et le corps de seringue en verre (12),

    dans lequel l'outil de formage (16) comprend

    - une partie de poignée ou de tige (28), adaptée pour être saisie et déplacée à l'aide de moyens moteurs appropriés,
    - un embout (32) adapté pour former ledit trou (20) sur le corps de seringue (12),
    - un corps d'embout (36), entre l'embout (32) et la partie de poignée (28), adapté pour réaliser ledit trou (20),
    - la partie de poignée (28), le corps d'embout (36) et l'embout (32) étant d'une seule pièce et alignés le long d'un axe d'extension et de rotation prédominant (X-X) du corps de seringue (12) et/ou de l'outil de formage (16),

    **caractérisé en ce que**

    - le corps d'embout (36), par rapport à un plan de section transversale perpendiculaire à l'axe d'extension prédominant (X-X), a une section transversale non circulaire, inscrite à l'intérieur du cercle maximum (40), ayant comme rayon la distance maximum entre un point (P) de ladite section transversale et l'axe d'extension prédominant (X-X), mesurée sur ledit plan de section transversale perpendiculaire à l'axe d'extension prédominant (X-X), de manière à avoir une section transversale globale inférieure à la section transversale du cercle maximum (40).

2. Appareil de formage (4) selon la revendication 1, dans lequel le corps d'embout (36), au niveau de sa section transversale maximum le long de l'axe d'extension prédominant X-X, a une section transversale inférieure à 85 % de la section transversale dudit cercle maximum (40) et dans lequel le corps d'embout (36), au niveau de sa section transversale maximum le long de l'axe d'extension prédominant X-X, a une section transversale inférieure à 70 % de la section transversale dudit cercle maximum (40).

3. Appareil de formage (4) selon l'une des revendications 1 à 2, dans lequel la section transversale du corps d'embout (36) identifie, par rapport au cercle maximum (40), au moins un évidement (44) adapté pour permettre le passage dudit liquide lubrifiant-réfrigérant.

4. Appareil de formage (4) selon la revendication 3, dans lequel le corps d'embout (36) présente, au niveau d'une section transversale le long de l'axe d'extension prédominant (X-X), une pluralité d'évidements (44) reliés par voie fluidique les uns aux autres de manière à créer un canal continu pour le passage dudit liquide lubrifiant-réfrigérant.

5. Appareil de formage (4) selon l'une des revendications 3 à 4, dans lequel les évidements (44) du corps d'embout (36) sont reliés les uns aux autres le long de l'axe d'extension prédominant (X-X), de manière à créer un canal continu pour le passage dudit liquide lubrifiant-réfrigérant le long du corps d'embout (36).

6. Appareil de formage (4) selon l'une quelconque des revendications 1 à 5, dans lequel ladite section transversale du corps d'embout (36) varie le long dudit axe d'extension prédominant (X-X) et dans lequel la section transversale du corps d'embout (36) se rétrécit le long dudit axe d'extension prédominant (X-X), en passant de la tige (28) à l'embout (32).

7. Appareil de formage (4) selon l'une des revendications 1 à 6, dans lequel ladite section transversale du corps d'embout (36) est un polygone régulier, inscrit à l'intérieur dudit cercle maximum (40), dans lequel ladite section transversale du corps d'embout (36) est une ligne brisée fermée, inscrite à l'intérieur dudit cercle maximum (40) et dans lequel la section transversale du corps d'embout (36) est curviligne, inscrite à l'intérieur dudit cercle maximum (40).

8. Appareil de formage (4) selon l'une des revendications 1 à 7, dans lequel ledit embout (32) est effilé par rapport à une partie de fixation de celui-ci au corps d'embout (36).

9. Appareil de formage (4) selon l'une des revendications 1 à 8, dans lequel ledit embout (32) a la même géométrie que le corps d'embout (36), par rapport à un plan de section transversale perpendiculaire à l'axe d'extension prédominant (X-X).

10. Outil de formage (16) profilé pour réaliser un trou (20) permettant de créer un cône de logement (8) dans le corps d'une seringue en verre (12), dans lequel ledit outil de formage (16) comprend

     - une partie de poignée ou de tige (28), adaptée pour être saisie et déplacée à l'aide de moyens moteurs appropriés,
     - un embout (32) adapté pour former ledit trou (20) sur le corps de seringue (12),
     - un corps d'embout (36), entre l'embout (32) et la partie de poignée (28), adapté pour réaliser ledit trou (20),
     - la partie de poignée (28), le corps d'embout (36) et l'embout (32) étant d'une seule pièce et alignés le long d'un axe d'extension et de rotation prédominant (X-X) du corps de seringue (12) et/ou de l'outil de formage (16),

     **caractérisé en ce que**

     - le corps d'embout (36), par rapport à un plan de section transversale perpendiculaire à l'axe d'extension prédominant (X-X), a une section transversale non circulaire, inscrite à l'intérieur du cercle maximum (40), ayant comme rayon la distance maximum entre un point (P) de ladite section transversale et l'axe d'extension prédominant (X-X), mesurée sur ledit plan de section transversale perpendiculaire à l'axe d'extension prédominant (X-X), de manière à avoir une section transversale globale inférieure à la section transversale du cercle maximum (40).

11. Outil de formage selon la revendication 10, ledit outil de formage (16), et en particulier l'embout (32) et le corps d'embout (36) dudit outil, étant constitué d'un matériau céramique dopé par un composé d'yttrium.

12. Outil de formage (16) selon la revendication 11, dans lequel ledit matériau céramique est du nitrure de si-

licium ($Si_3N_4$) dopé à l'oxyde d'yttrium ($Y_2O_3$), dans lequel de préférence l'oxyde d'yttrium est contenu dans le nitrure de silicium en quantités comprises entre 3 % et 7 % ou entre 4 % et 6 % en poids, ou ledit matériau céramique est du nitrure de silicium contenant de l'oxyde d'yttrium et de l'alumine ($Al_2O_3$) en quantité combinée comprise entre 7 % et 13 %, ou entre 8 % et 12 % en poids.

13. Procédé de fabrication d'une seringue en verre pour substances médicales, munie d'un cône de logement (8) d'une aiguille, comprenant les étapes consistant à :

     - préparer un corps de seringue en verre (12) muni d'une paroi latérale (23) destinée à délimiter ledit trou (20) dans le cône de logement (8),
     - fournir un outil de formage (4) selon l'une des revendications 1 à 9,
     - faire tourner le corps de seringue (12) et/ou l'outil de formage (16) autour de l'axe d'extension prédominant (X-X),
     - former ladite paroi latérale (23) du corps de seringue (12), après avoir aligné l'axe d'extension prédominant (X-X) de l'outil de formage (16) avec un axe de symétrie (S-S) du trou (20) à réaliser.

14. Seringue réalisée avec l'outil de formage (16) selon la revendication 11, **caractérisée en ce que** ladite seringue (12) ne contient pas de tungstène et contient une quantité résiduelle d'yttrium comprise entre 0,5 ng et 1 ng.

15. Seringue (12) selon la revendication 14, remplie d'une des substances actives suivantes : tocilizumab, darbépoétine alfa, bévacizumab, interféron bêta 1-alpha, interféron bêta 1b, toxine onabotulinique A, exénatide, imiglucérase, certolizumab pégol, acétate de glatiramère, sécukinumab, triptoréline, dupilumab, étanercept, époétine, cétuximab, aflibercept, follitropine bêta, tériparatide, vaccins contre le papillomavirus, glucagon, FSH - hormone folliculo-stimulante, trastuzumab, insuline lispro, insuline, adalimumab, dibotermine alfa, interféron alfa 2a, palipéridone, pembrolizumab, anakinra, facteur VIII - facteur antihémophilique, insuline glargine, énoxaparine, ranibizumab, alemtuzumab, rituximab, ténectéplase, toxine botulique de type A, époétine bêta, pegfilgrastim, filgrastim, somatropine, insuline asparte, heptagone alfa activé, romiplostim, pégaspargase, nivolumab, abatacept, choriogonadotropine alfa, interféron alfa 2a pégylé, pertuzumab, interféron bêta 1a pégylé, vaccins contre la pneumonie à streptocoque, dénosumab, infliximab, altéplase, golimumab, basiliximab, éculizumab, ustékinumab, palivizumab, atézolizumab, insuline dégludec, ibalizumab, liraglutide, ranibizumab, omalizulab et pégaspargase.

FIG.1a

FIG.1b

EP 3 929 165 B1

FIG.2c    FIG.3c    FIG.4c    FIG.5c

FIG.2a    FIG.2b    FIG.3a    FIG.3b    FIG.4a    FIG.4b    FIG.5a    FIG.5b

EP 3 929 165 B1

FIG.6c

FIG.7c

FIG.8c

FIG.9c

FIG.6a   FIG.6b   FIG.7a   FIG.7b   FIG.8a   FIG.8b   FIG.9a   FIG.9b

EP 3 929 165 B1

FIG.14c FIG.13c FIG.12c FIG.11c FIG.10c

FIG.14b FIG.14a FIG.13b FIG.13a FIG.12b FIG.12a FIG.11b FIG.11a FIG.10b FIG.10a

**EP 3 929 165 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019144326 A1